# EUROPÄISCHE PATENTSCHRIFT

(11) **EP 0 392 032 B1**
(45) Veröffentlichungstag und Bekanntmachung des Hinweises auf die Patenterteilung: **25.01.1995**
(21) Anmeldenummer: 89106275.4
(22) Anmeldetag: 10.04.1989
(51) Int. Cl.: A61N 1/37

(54) **Implantierbares medizinisches Gerät mit Mitteln zum telemetrischen Übertragen von Daten**
Medical implantable apparatus with telematic data transmission means
Appareil médical implantable comportant des moyens de transmission télémétrique de données

(43) Veröffentlichungstag der Anmeldung: 17.10.1990
(73) Patentinhaber: Pacesetter AB, 171 95 Solna (SE)
(72) Erfinder: Vock, Josef, Dipl.-Ing., S-163 60 Spanga (SE); Engström, Jan, Dipl.-Ing., S-163 55 Spanga (SE); Ekwall, Christer, Dipl.-Ing., S-163 57 Spanga (SE)
(74) Vertreter: Lettström, Richard Wilhelm

(56) Entgegenhaltungen:
- EP-A- 0 225 839
- US-A- 4 571 589
- US-A- 4 705 043
- A. Bruce Carlson, "Communication systems", McGraw Hill Book Company, 1986, Seiten 470 und 471

## Beschreibung

Die Erfindung betrifft ein in den Körper eines Lebewesens implantierbares medizinisches Gerät mit Mitteln zum Detektieren von physiologischen Ereignissen und/oder mit Mitteln zum Stimulieren von physiologischen Vorgängen, mit einer Steuerlogik, an welche die Mittel zum Detektieren und/oder die Mittel zum Stimulieren angeschlossen sind, und mit Mitteln zum telemetrischen Übertragen von den logischen Zustand der Steuerlogik betreffenden Daten zu einem externen Empfänger, wobei die Mittel zum Übertragen von Daten mit der Steuerlogik verbunden sind.

Bei derartigen Geräten, z.B. Herzschrittmachern oder Defibrillatoren, hängt der logische Zustand der Steuerlogik u.a. davon ab, ob ein physiologisches Ereignis detektiert bzw. ein physiologischer Vorgang stimuliert wurde. Stellt man die physiologischen Funktionen des Lebewesens, bezüglich derer Ereignisse detektiert und Vorgänge stimuliert werden, als Funktion der Zeit, also beispielsweise als Elektrokardiogramm, und parallel dazu in korrekter zeitlicher Zuordnung die den logischen Zustand der Steuerlogik betreffenden Daten dar, ist es möglich, das Zusammenspiel des Gerätes mit dem Körper des Lebewesens zu beurteilen. Dies ist im Falle von Geräten der eingangs genannten Art infolge der telemetrischen Übertragung der Daten auf nicht-invasivem Weg möglich.

Ein Gerät der eingangs genannten Art ist in der EP-A-0 120 250 beschrieben. Es handelt sich hierbei um einen Herzschrittmacher, der Daten bezüglich eines vorhergehenden Herz- bzw. Schrittmacher-Zyklus speichert und die Daten mit Verzögerung zu dem Empfänger überträgt, und zwar dann, wenn ein Ereignis eintritt, das einen Zyklus abschliesst. Die Datenübertragung erfolgt also mit einer Verzögerung, die der Dauer eines Zyklus entspricht. Um die übertragenen Daten in korrekter zeitlicher Zuordnung zu einem Elektrokardiogramm des Lebewesens darstellen zu können, ist es somit erforderlich, die Darstellung des Elektrokardiogramms zu verzögern, was mit erheblichem Aufwand verbunden ist. Ausserdem erfolgt eine Übertragung der Daten bezüglich jedes Zyklus nur einmal. Die Gefahr von Datenverlusten ist demzufolge gross. Eine wirksame Korrektur von Datenübertragungsfehlern in dem Empfänger ist nur insoweit möglich, als die Annahme von offensichtlich unsinnigen Daten unterbunden werden kann.

Ein weiteres Gerät der eingangs genannten Art ist in der US-A 4,374,382 beschrieben. Bei diesem Gerät bewirkt die Detektion eines physiologischen Ereignisses bzw. die Stimulation eines physiologischen Vorganges die Bildung und Übertragung eines dem jeweiligen Ereignis bzw. Vorgang entsprechenden Codes. Die Datenübertragung erfolgt also quasi-gleichzeitig mit dem Auftreten des Ereignisses bzw. Vorganges. Allerdings erfolgt auch hier die Datenübertragung pro Ereignis oder Vorgang nur ein einziges Mal. Auch hier ist also die Gefahr von Datenverlusten gross und eine wirksame Fehlerkorrektur bezüglich der übertragenen Daten unmöglich.

Zur Vermeidung von Datenverlusten bei Datenübertragung ist bekannt, eine mehrfache Übertragung des zu sichernden Datums (siehe A. Bruce Carlson, "Communication systems", McGraw Hill Book Company, 1986, Seiten 470 und 471) bzw. der zu sichernden Nachricht vorzusehen, wobei dann die "richtige" Nachricht durch Majoritätsentscheidung erhalten wird.

Der Erfindung liegt die Aufgabe zugrunde, ein Gerät der eingangs genannten Art so auszubilden, dass die Datenübertragung mit hoher Zuverlässigkeit möglich ist, die Gefahr von Datenverlusten vermindert ist, und die Voraussetzung für eine wirksame Korrektur von Datenübertragungsfehlern geschaffen ist.

Nach der Erfindung wird diese Aufgabe durch die Merkmale des Anspruchs 1 gelöst. Infolge der fortlaufenden Datenübertragung besteht also keine Verknüpfung der Datenübertragung mit der Detektion von physiologischen Ereignissen bzw. der Stimulation von physiologischen Vorgängen. Somit können die Daten bezüglich eines bestimmten logischen Zustandes der Steuerlogik mehrfach übertragen werden, was die Zuverlässigkeit der Datenübertragung erheblich steigert und gleichzeitig die Voraussetzung für eine Korrektur von Datenübertragungsfehlern bietet. Ausserdem ist infolge der Übertragung der jeweils aktuellen Daten eine Verzögerung der Datenübertragung gegenüber dem jeweiligen Zustand der Steuerlogik vermieden. Die Datenübertragung kann sowohl analog als auch digital erfolgen. Im Falle einer analogen Datenübertragung kann z.B. Frequenz-Modulation Anwendung finden. Es ist dann jedem möglichen logischen Zustand der Steuerlogik eine bestimmte Frequenz zugeordnet, mit der die Mittel zum Übertragen von Daten ein Trägersignal bei Auftreten des entsprechenden logischen Zustandes modulieren. In diesem Fall erfolgt eine völlig kontinuierliche Datenübertragung, während im Falle einer digitalen Datenübertragung in der Regel nur eine quasikontinuierliche Datenübertragung möglich ist, die bei ausreichend rascher Folge der einzelnen Datenübertragungen aber die gleichen Vorteile wie eine kontinuierliche Datenübertragung bietet, jedoch technisch weniger aufwendig ist.

Bei einer bevorzugten Ausführungsform der Erfindung ist ein Taktgenerator vorgesehen, der die Mittel zum Übertragen von Daten zu kontinuierlich aufeinanderfolgenden Zeitpunkten zur Datenübertragung aktiviert, wobei die Mittel zum Übertragen von Daten die zu dem Zeitpunkt der Datenübertragung jeweils aktuellen Daten übertragen. Die Datenübertragung erfolgt somit wieder unabhängig von detektierten physiologischen Ereignissen oder stimulierten physiologischen Vorgängen, da ein Taktgenerator zur Aktivierung der Mittel zum Übertragen von Daten zu vorzugsweise periodisch aufeinanderfolgenden Zeitpunkten vorgesehen ist. In den durch die maximale Datenübertragungsgeschwindigkeit der Mittel zum Übertragen von Daten und die pro Datenübertragung zu übertragende Datenmenge gesteckten Grenzen können die Mittel zum Übertragen von Daten mittels des Taktgenerators beliebig oft aktiviert werden. Es erfolgt also eine quasi-kontinuierliche Datenübertragung, wobei bei geeigneter Wahl der Dauer der zwischen aufeinanderfolgenden Zeitpunkten liegenden Zeitintervalle während der Dauer eines logischen Zustandes die Mittel zum Übertragen von Daten mehrfach aktiviert werden. Die einem bestimmten logischen Zustand entsprechenden Daten werden somit mehrfach übertragen, so dass Datenverluste praktisch ausgeschlossen sind. Infolge der mehrfachen Übertragung der Daten ist ausserdem die Voraussetzung für eine wirksame Korrektur von Datenübertragungsfehlern geschaffen, da z.B. die Möglichkeit besteht, den Empfänger derart auszubilden, dass er nur solche Daten akzeptiert, die mehrfach aufeinanderfolgend unverändert übertragen wurden.

Falls das Gerät eine sich aufeinanderfolgenden Gerätezyklen vollziehende zyklische Arbeitsweise besitzt, ist gemäss einer besonders vorteilhaften Variante der Erfindung vorgesehen, dass der Taktgenerator die Mittel zum Übertragen von Daten während eines Gerätezyklus mehrfach aktiviert. Durch diese Massnahme ist selbst dann, wenn sich während eines Gerätezyklus der logische Zustand der Steuerlogik rasch ändert, gewährleistet, dass die Übertragung der entsprechenden Daten mit hoher Zuverlässigkeit erfolgt. Grundsätzlich ist es auch denkbar, dass der Taktgenerator die Mittel zum Übertragen von Daten während eines Gerätezyklus nur einmal aktiviert; dies ist jedoch nicht beansprucht und nur dann sinnvoll, wenn die zu übertragenden Daten logischen Zuständen der Steuerlogik entsprechen, die in der Regel über mehrere Gerätezyklen unverändert bleiben.

Obwohl im Rahmen der Erfindung eine parallele Datenübertragung nicht ausgeschlossen ist, ist gemäss einer Variante der Erfindung vorgesehen, dass die Mittel zum Übertragen von Daten die Daten seriell übertragen. In Anbetracht des Umstandes, dass die Datenübertragung nicht drahtgebunden, sondern telemetrisch erfolgt, lässt sich auf diese Weise der technische als auch finanzielle Aufwand für das Gerät ganz erheblich mindern.

Gemäss einer Variante der Erfindung ist das Gerät als Herzschrittmacher ausgebildet, wobei die Mittel zum Detektieren von physiologischen Ereignissen die elektrische Aktivität des Herzens überwachen und die Mittel zum Stimulieren von physiologischen Vorgängen das Herz stimulieren. In diesem Falle kann in vorteilhafter Weise vorgesehen sein, dass die Mittel zum Detektieren von physiologischen Ereignissen ein der zugehörigen physiologischen Funktion, im Falle eines Herzschrittmachers also ein der elektrischen Aktivität des Herzens entsprechendes elektrisches Signal bilden, welches einem Analog/Digital-Wandler zugeführt ist, dessen digitales Ausgangssignal den Mitteln zum Übertragen von Daten zugeführt ist, die dieses zu dem Empfänger übertragen. Da also neben den Mitteln zum Übertragen von Daten auch die Mittel zum Detektieren von physiologischen Ereignissen und damit Mittel zur Bildung des der jeweiligen physiologischen Funktion entsprechenden elektrischen Signals ohnehin vorhanden sind, können mit dem erfindungsgemässen Gerät auf einfache Weise der physiologischen Funktion entsprechende Daten, im Falle eines Herzschrittmacher ein intrakardiales Elektrogramm (IEKG), gewonnen und zu dem Empfänger übertragen werden. Ausserdem kann vorgesehen sein, dass den Mitteln zum Übertragen von Daten ausser den den logischen Zustand der Steuerlogik betreffenden Daten weitere den Betriebszustand des Geräts betreffende Daten zugeführt sind, die die Mittel zum Übertragen von Daten zu dem Empfänger übertragen. Anhand derartiger Daten ist es auf einfache Weise möglich, die ordnungsgemässe technische Funktion des implantierten Gerätes zu überprüfen. In diesem Zusammenhang ist gemäss einer Variante der Erfindung vorgesehen, dass die Mittel zum Übertragen von Daten sowohl die dem logischen Zustand der Steuerlogik entsprechenden Daten als auch die digitalen Ausgangssignale des Analog/Digital-Wandlers oder die den Betriebszustand des Gerätes betreffenden Daten in Form eines einzigen Datenwortes übertragen, wobei jeweils eine zusammenhängende bit-Folge des Datenwortes die dem logischen Zustand der Steuerlogik entsprechenden Daten und die digitalen Ausgangssignale des Analog/Digital-Wandlers enthält. Durch diese Massnahme ist sichergestellt, dass die korrekte zeitliche Zuordnung der dem logischen Zustand der Steuerlogik entsprechenden Signale zu dem der elektrischen Aktivität des Herzen entsprechenden Signal, also z. B. dem IEKG, ohne besondere Massnahmen bereits bei der Übertragung der Daten gewährleistet ist.

Ein Ausführungsbeispiel der Erfindung ist in den beigefügten Figuren dargestellt. Es zeigen:
- FIG 1: ein Blockschaltbild eines erfindungsgemäss aufgebauten Herzschrittmachers, und
- FIG 2 u. 3: die Struktur der übertragenen Daten.

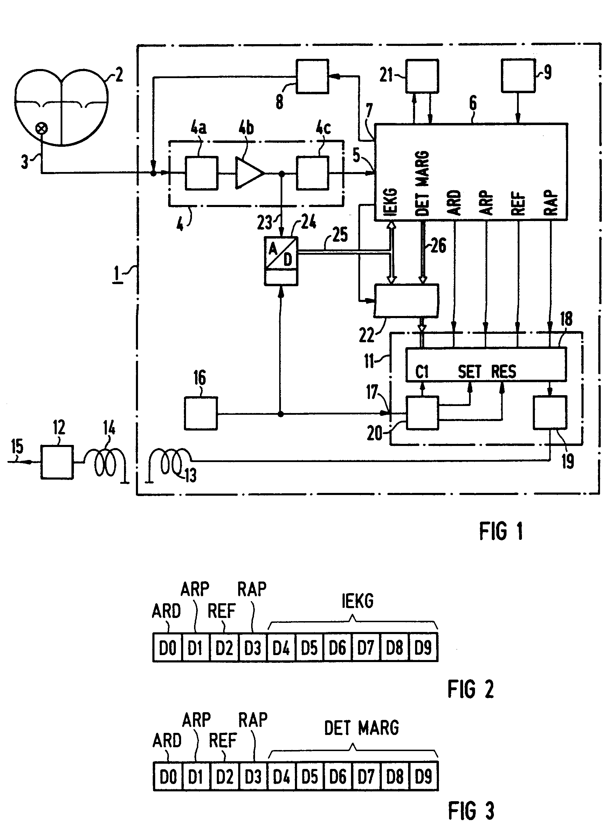

In FIG 1 ist ein insgesamt mit (1) bezeichneter Herzschrittmacher dargestellt, der im VVI-Mode arbeitet. Demgemäss steht der Herzschrittmacher (1) über eine in das Ventrikel eines schematisch angedeuteten Herzens (2) eines Lebewesens eingesetzte Elektrode (3) mit dem Herzen (2) in Verbindung.

Die Elektrode (3) dient einerseits dazu, ein der elektrischen Aktivität des Herzens (2) entsprechendes Signal zu erfassen und dem Herzschrittmacher (1) zuzuführen. Dort gelangt das Signal zu einer schematisch angedeuteten Detektorschaltung (4), in der das Signal ein EKG-Filter (4a) und einen Verstärker (4b) durchläuft und einer Pegelmesseinheit (4c) zugeführt wird, die bei Auftreten eines Ereignisses mit einer einem natürlichen Herzschlag entsprechenden Mindestamplitude und/oder einer bestimmten, für natürliche Herzschläge typischen Steilheit in dem Ausgangssignal des Verstärkers (4c) ein die Detektion eines natürlichen Herzschlages anzeigendes digitales Signal abgibt. Der Ausgang der Detektorschaltung (4) ist mit einem Eingang (5) einer Steuerlogik (6) verbunden, die anhand der von der Detektorschaltung (4) eingehenden digitalen Signale unter Heranziehung gespeicherter Daten entscheidet, ob und wann eine Stimulation des Herzens (2) erfolgen soll. Ist eine Stimulation des Herzens (2) erforderlich, gibt die Steuerlogik (6) über einen Ausgang (7) zum entsprechenden Zeitpunkt ein Signal an einen Stimulationspuls-Generator (8), der zur Erzeugung eines Stimulationspulses bestimmter Amplitude und Dauer dient.

Die Elektrode (3) dient andererseits dazu, dem Herzen (2) Stimulationspulse zuzuführen. Der Ausgang des Stimulationspuls-Generators (8) ist daher mit der Elektrode (3) verbunden.

Alle für die Funktion der Steuerlogik (6) erforderlichen Zeitsignale werden ausgehend von dem Signal eines Taktgenerators (9), z. B. eines Quarz-Oszillators, gewonnen, der mit einem Takteingang der Steuerlogik (6) verbunden ist. Ausserdem ist eine mit der Steuerlogik (6) verbundene Timerschaltung (21) vorhanden, die zur Messung einer definierten Zeitdauer dient und von der Steuerlogik (6) gestartet wird, sobald ein Stimulationspuls mittels des Stimulationspuls-Generators (8) abgegeben wird. Solange die definierte Zeitdauer läuft, die einer maximal zulässigen Folgefrequenz der Stimulationspulse entspricht, unterbindet die Steuerlogik (6) die Abgabe eines weiteren Stimulationspulses. Auf diese Weise ist vermieden, dass, z.B. im Falle von Störungen des Taktgenerators (9), eine Folgefrequenz der Stimulationspulse auftritt, die physiologisch nicht zulässig ist und den Patienten gefährden würde.

Die Steuerlogik (6) weist eine Reihe von Ausgängen auf, die in FIG 1 die Bezeichnung REF, ARD, ARP und RAP tragen und jeweils entweder den Zustand logisch "1" oder logisch "0" annehmen können. Im einzelnen besitzt der Ausgang REF den Zustand logisch "1" solange die technische Refraktärzeit läuft. Der Ausgang ARD nimmt den Zustand logisch "1" an, sobald ein natürlicher Herzschlag detektiert wird und behält diesen Zustand bei, solange die absolute technische Refraktärzeit läuft. Der Ausgang ARP nimmt den Zustand logisch "1" an, sobald ein Stimulationspuls abgegeben wird und behält diesen Zustand für die Dauer der absoluten Refraktärzeit bei. Der Ausgang RAP nimmt immer dann, wenn die Timerschaltung (21) gestartet wird, für die Dauer der definierten Zeitdauer den Zustand logisch "1" an, so dass überprüft werden kann, ob die Timerschaltung (21) korrekt arbeitet.

Einzelheiten bezüglich der in der Herzschrittmacher-Technologie gängigen Begriffe "technische Refraktärzeit" und "absolute technische Refraktärzeit" können der einschlägigen Literatur entnommen werden (siehe z.B. Druckschrift "DIALOG-Schrittmacher 728" - Gebrauchsanweisung, Oktober 1986, Siemens-Elema AB, Solna, Schweden).

Es wird somit deutlich, dass an den erwähnten Ausgängen der Steuerlogik (6) Daten zur Verfügung stehen, die dem momentanen logischen Zustand der Steuerlogik (6) entsprechen. Diese Daten können, beispielsweise in geeigneter Form und in korrekter zeitlicher Zuordnung zu einem Elektrodkardiogramm dargestellt, wichtige Aufschlüsse über das Zusammenspiel des Herzschrittmachers (1) mit dem Herzen (2) geben.

Der Herzschrittmacher (1) besitzt daher eine mit den erwähnten Ausgängen der Steuerlogik (6) verbundene Telemetrieschaltung (11), die es gestattet, die dem logischen Zustand der Steuerlogik (6) entsprechenden Daten von dem in den Körper eines Lebewesens implantierten Herzschrittmacher (1) zu einem getrennten Empfänger (12) auf nicht-invasivem Wege zu übertragen.

Die Übertragung der Daten erfolgt auf induktivem Wege, d.h. der Ausgang der Telemetrieschaltung (11) ist mit einer Sendespule (13) verbunden, in deren Nähe eine mit dem Eingang des Empfängers (12) verbundene Empfangsspule (14) derart angeordnet wird, dass diese mit der Sendespule (13) induktiv gekoppelt ist.

Vom Ausgang (15) des Empfängers (12) gelangen die Daten zu einem EKG-Rekorder oder einem anderen Gerät, das zur Auswertung bzw. Darstellung der Daten geeignet ist.

Wie aus der FIG 1 ersichtlich ist, ist ein Taktgenerator (16) vorgesehen, der ein Taktsignal erzeugt, das zu einem Takteingang (17) der Telemetrieschaltung (11) gelangt. Bei dem Taktgenerator (16) handelt es sich um einen Oszillator, der ein Signal konstanter Periodendauer abgibt. Geeignete Schaltungen sind dem Fachmann bekannt. Das an dem Takteingang (17) der Telemetrieschaltung (11) anliegende Signal dient dazu, die Telemetrieschaltung (11) zu kontinuierlich, d.h. periodisch aufeinanderfolgenden Zeitpunkten zur Übertragung der dem logischen Zustand der Steuerlogik (6) entsprechenden Daten zu aktivieren.

Die Telemetrieschaltung (11) enthält ein Schieberegister (18) mit parallelen Eingängen, wobei jeweils einer der genannten Ausgänge der Steuerlogik (6) mit einem Eingang des Schieberegisters (18) verbunden ist, und einem seriellen Ausgang, der über eine Modulatorschaltung (19), z.B. einen FSK-Modulator, mit der Sendespule (13) verbunden ist. Das der Telemetrieschaltung (11) über den Takteingang (17) zugeführte Signal wird mittels einer Taktaufbereitungsschaltung (20) derart aufbereitet, dass es das Schieberegister (18) in einer solchen Weise steuert, dass dieses die unmittelbar vor Beginn einer Datenübertragung an seinen parallelen Eingängen vorhandenen logischen Zustände bzw. Daten speichert, die dann der Modulatorschaltung (19) seriell zugeführt werden. Die Taktaufbereitungsschaltung (20) erzeugt also die zum Betrieb des Schieberegisters (18) erforderlichen Parallel-Eingabe-Taktimpulse, Rückstell-Taktimpulse und Schiebe-Taktimpulse, die entsprechenden Engängen des Schieberegisters (18) zugeführt wird, die mit SET, RES und C1 bezeichnet sind. Das Ausgangssignal der Modulatorschaltung (19) gelangt über die Sendespule (13) und die Empfangsspule (14) zu dem Empfänger (12) und wird dort in geeigneter Weise demoduliert und steht am Ausgang (15) des Empfängers (12) als serieller Datenstrom zur Verfügung.

Es wird somit deutlich, dass bei dem erfindungsgemässen Herzschrittmacher (1) die Übertragung der dem logischen Zustand der Steuerlogik (6) entsprechenden Daten unabhängig von dem Auftreten irgendwelcher Ereignisse, z.B. der Detektion eines natürlichen Herzschlages oder der Abgabe eines Stimulationspulses, quasi-kontinuierlich zu periodisch aufeinanderfolgenden Zeitpunkten erfolgt. Dabei werden jeweils die zum Beginn einer Datenübertragung aktuellen Daten übertragen .

Bekanntlich besitzt ein Herzschrittmacher eine sich in aufeinanderfolgenden Gerätezyklen vollziehende zyklische Arbeitsweise, wobei die kürzestmögliche Zyklusdauer der Dauer der technischen Refraktärzeit entspricht und die längstmögliche Zyklusdauer durch die Länge eines Zeitintervalles bestimmt ist, dessen Dauer sich aus der Frequenz ergibt, mit der der Herzschrittmacher das Herz im Bedarfsfalle stimuliert. Die Gesamtdauer der technischen Refraktärzeit liegt gewöhnlich in der Grössenordnung von 200 bis 500 ms. Die Taktfrequenz des Taktgenerators (16) ist derart gewählt, dass während eines Gerätezyklus, d.h. während des Ablaufs der jeweils gewählten technischen Refraktärzeit mehrere Datenübertragungen erfolgen. Geht man davon aus, dass im Interesse einer ausreichend zuverlässigen Datenübertragung während eines Gerätezyklus wenigstens 30 Datenübertragungen erfolgen sollen, müssen unter Zugrundelegung einer technischen Refraktärzeit von 240 ms die Datenübertragungen in Abständen von höchstens 8 ms aufeinanderfolgen.

Wie aus der FIG 1 ersichtlich ist, gelangt das gefilterte und verstärkte Signal der Elektrode (3) zu einem Digital/Analog-Wandler (24) mit einer Auflösung von beispielsweise 6 bit, der seine Taktsignale von dem Taktgenerator (16) erhält. Das verstärkte und gefilterte Signal entspricht einem intrakardialen Elektrogramm (IEKG) und wird von dem Digital/Analog-Wandler (24) in digitalisierter Form über einen schematisch angedeuteten, 6 bit breiten Datenbus (25) zum einen zu einer elektronischen Schalteinrichtung (22) und zum anderen zu einem mit IEKG bezeichneten Eingang der Steuerlogik (6) geleitet. Wird die Schalteinrichtung (22) über eine Steuerleitung (23) mittels der Steuerlogik (6) in geeigneter Weise betätigt, gelangen die Ausgangsdaten des Analog/Digital-Wandlers (24) zu der Telemetrieschaltung (11), wobei jeweils eine Leitung des Datenbusses (25) mit einem Eingang des Schieberegisters (18) verbunden ist, das insgesamt eine Breite von 10 bit aufweist. Diese überträgt die dem IEKG entsprechenden Daten gemeinsam mit den dem logischen Zustand der Steuerlogik (6) entsprechenden Daten.

Dies geschieht gemäss FIG 2 in einem einzigen Datenwort mit den 10 bit D0 bis D9, wobei eine zusammenhängende Folge von 6 bit des Datenwortes die dem IEKG entsprechenden digitalen Ausgangssignale des Analog/Digital-Wandlers (24) und eine zusammenhängende Folge von 4 bit Datenwortes die dem logischen Zustand der Steuerlogik (6) entsprechenden Daten enthält.

Die Steuerlogik (6) errechnet aus den ihr zugeführten digitalen Ausgangssignalen des Analog/Digital-Wandlers (24) und der Mindestamplitude, die das der elektrischen Aktivität des Herzens (2) entsprechende Signal aufweisen muss, um in der Pegelmesseinheit (4c) zur Detektion eines natürlichen Herzschlages zu führen, für jeden detektierten natürlichen Herzschlag das sogenannte Detektionsmarginal. Das Detektionsmarginal gibt an, um welches Mass die Amplitude des der elektrischen Aktivität des Herzens (2) entsprechenden Signals bei der Detektion eines natürlichen Herzschlages die Mindestamplitude übersteigt. Anhand des Detektionsmarginals, das eine wichtige Grösse zur Beurteilung des Betriebszustandes des Herzschrittmachers (1) darstellt, kann also festgestellt werden, ob der eingestellte Wert der Mindesamplitude so gewählt ist, dass alle auftretenden natürlichen Herzschläge mit hoher Wahrscheinlichkeit detektiert werden können. Ist das Detektionsmarginal zu gering, kann eine entsprechende Korrektur des Wertes der Mindestamplitude erfolgen. Dem Detektionsmarginal entsprechende Daten stehen an einem DET MARG bezeichneten Ausgang der Steuerlogik (6) zur Verfügung und sind über einen schematisch angedeuteten 6 bit breiten Datenbus (26) der Schalteinrichtung (22) zugeführt. Betätigt die Steuerlogik (6) die Schalteinrichtung (22) über die Steuerleitung (23) in geeigneter Weise, gelangen anstelle der Ausgangsdaten des Analog/Digital-Wandlers (24) die dem Detektionsmarginal entsprechenden digitalen Daten zu der Telemetrieschaltung (11) bzw. deren Schieberegister (18). Die Telemetrieschaltung (11) überträgt dann die dem Detektionsmarginal entsprechenden Daten gemeinsam mit den dem logischen Zustand der Steuerlogik (6) entsprechenden Daten. Dies geschieht gemäss FIG 3 wieder in einem einzigen Datenwort mit den 10 bit D0 bis D9, wobei eine zusammenhängende Folge von 6 bit des Datenwortes die dem Detektionsmarginal entsprechenden Daten und eine zusammenhängende Folge von 4 bit des Datenwortes die dem logischen Zustand der Steuerlogik (6) entsprechenden Daten enthält.

Es besteht auch die Möglichkeit, dass die Steuerlogik (6) die Schalteinrichtung (22) in einer solchen Weise betätigt, dass diese als Multiplexer wirkt und die Telemetrieschaltung (11) abwechselnd ein Datenwort gemäss FIG 2 und ein Datenwort gemäss FIG 3 usw. überträgt.

Zusätzlich zu den Daten werden unter Umständen in nicht dargestellter und an sich bekannter Weise Synchronisierungsimpulse und Stop-bits zu dem Empfänger (12) übertragen.

Im Falle des beschriebenen Ausführungsbeispieles ist ein besonderer Taktgenerator (16) vorgesehen, der die Telemetrieschaltung (11) zur Datenübertragung aktiviert. Die Funktion des Taktgenerators (16) kann aber auch von dem der Steuerlogik (6) zugeordneten Taktgenerator (9) wahrgenommen werden, wenn das Signal des Taktgenerators (9) in geeigneter Weise aufbereitet und dem Takteingang (18) der Telemetrieschaltung (11) zugeführt wird. In Fällen, in denen ein bidirektionaler Datenverkehr zwischen dem Herzschrittmacher (1) und einem externen Gerät stattfindet, dies ist z. B. im Falle von programmierbaren Herzschrittmachern der Fall, besteht auch die Möglichkeit, dass sich der Taktgenerator in dem externen Gerät befindet und die die Telemetrieschaltung (11) des Herzschrittmachers (1) zur Übertragung von Daten aktivierenden Signale telemetrisch zu der Telemetrieschaltung (11) übertragen werden. In diesem Falle können der Steuerlogik (6) zur Betätigung der Schalteinrichtung (22) dienende Signale telemetrisch zugeführt werden.

Die im Falle des beschriebenen Ausführungsbeispiels zur telemetrischen Übertragung bestimmten Daten sind nur beispielhaft zu verstehen. Es können auch weitere oder andere Daten übertragen werden.

Die Erfindung ist vorstehend zwar nur am Beispiel eines implantierbaren Herzschrittmachers beschrieben. Sie kann jedoch auch bei anderen implantierbaren medizinischen Geräten Anwendung finden.

## Patentansprüche

1. In den Körper eines Lebewesens implantierbares medizinisches Gerät (1),
mit Mitteln (3,4,4a,4b,4c) zum Detektieren von physiologischen Ereignissen und/oder mit Mitteln (3,8) zum Stimulieren von physiologischen Vorgängen,
mit einer mit Ausgängen versehenen Steuerlogik (6), an welche die Mittel (3,4,4a,4b,4c) zum Detektieren und/oder die Mittel (3,8) zum Stimulieren angeschlossen sind,
und mit Mitteln (11,13) zum telemetrischen Übertragen von den logischen Zustand der Steuerlogik (6) betreffenden Daten zu einem externen Empfänger (12), wobei die Mittel (11,13) zum Übertragen von Daten mit der Steuerlogik (6) verbunden sind, **dadurch gekennzeichnet,** daß
die Mittel (11,13) zum Übertragen von Daten Mittel(17,18,20) zum Erfassen der Daten an den Ausgängen der Steuerlogik (6) enthalten, wobei diese Daten dem momentanen logischen Zustand der Steuerlogik (6) entsprechen, und wobei die Mittel (17,18,20) zum Erfassen der Daten im wesentlichen gleichzeitig mit den Mitteln (11,13) zum Übertragen aktiviert werden,
die Mittel (11,13) zum Übertragen zusätzlich einen Taktgenerator (16) aufweist, welcher die Mittel zum Übertragen (11,13) und die Mittel (17,18,20) zum Erfassen der Daten zu den durch den Taktgenerator (16) festgelegten und periodisch aufeinanderfolgenden Zeitpunkten repetitiv aktivieren, wobei die Frequenz des Taktgenerators (16) höher als die Umschlagfrequenz des logischen Zustands der Steuerlogik (6) ist.

2. Gerät nach Anspruch 1, welches eine sich in aufeinanderfolgenden Gerätezyklen vollziehende zyklische Arbeitsweise besitzt, **dadurch gekennzeichnet,** daß der Taktgenerator (16) die Mittel(11,13) zum Übertragen von Daten während eines Gerätezyklus mehrfach aktiviert.

3. Gerät nach einem der Ansprüche 1 bis 2, **dadurch gekennzeichnet,** daß die Mittel (11,13) zum Übertragen von Daten die Daten seriell übertragen.

4. Gerät nach einem der Ansprüche 1 bis 3, **dadurch gekennzeichnet,** daß das Gerät (1) als Herzschrittmacher ausgebildet ist, wobei die Mittel (3,4,4a,4b,4c) zum Detektieren von physiologischen Ereignissen die elektrische Aktivität des Herzens (2) überwachen und die Mittel zum Stimulieren (3,8) von physiologischen Vorgängen das Herz (2) stimulieren.

5. Gerät nach einem der Ansprüche 1 bis 4, **dadurch gekennzeichnet,** daß die Mittel (3,4,4a,4b,4c) zum Detektieren von physiologischen Ereignissen ein der zugehörigen physiologischen Funktion entsprechendes elektrisches Signal bilden, welches einem Analog/Digital-Wandler (24) zugeführt ist, dessen digitales Ausgangssignal den Mitteln (11,13) zum Übertragen von Daten zugeführt ist, die dieses zu dem Empfänger (12) übertragen.

6. Gerät nach einem der Ansprüche 1 bis 5, **dadurch gekennzeichnet,** daß den Mitteln (11,13) zum Übertragen von Daten außer den logischen Zustand der Steuerlogik (6) betreffenden Daten weitere den Betriebszustand des Gerätes (1) betreffende Daten zuführbar sind, die die Mittel (11,13) zum Übertragen von Daten zu dem Empfänger (12) übertragen.

7. Gerät nach Anspruch 5 oder 6, **dadurch gekennzeichnet,** daß die Mittel (11,13) zum Übertragen von Daten sowohl die dem logischen Zustand der Steuerlogik (6) entsprechenden Daten als auch die digitalen Ausgangssignale des Analog/Digital-Wandlers (24) oder die den Betriebszustand des Gerätes (1) betreffenden Daten in Form eines einzigen Datenwortes (D0 bis D9) übertragen, wobei jeweils eine zusammenhängende bit-Folge (D0 bis D3 bzw. D4 bis D9) des Datenwortes (D0 bis D9) die dem logischen Zustand der Steuerlogik (6) entsprechenden Daten und die digitalen Ausgangssignale des Analog/Digital-Wandlers (24) oder die den Betriebszustand des Gerätes (1) betreffenden Daten enthält.

## Claims

1. Medical apparatus (1) which can be implanted into the body of a living organism,
having means (3,4,4a,4b,4c) for the detection of physiological events and/or having means (3,8) for the stimulation of physiological processes,
having a control logic (6) which is provided with outputs and to which the detecting means (3,4,4a,4b,4c) and/or the stimulating means (3,8) are connected,
and having means (11,13) for the telemetric transmission of data concerning the logic state of the control logic (6) to an external receiver (12), the means (11,13) for the transmission of data being connected to the control logic (6), characterized in that
the means (11,13) for the transmission of data include means (17,18,20) for the acquisition of the data at the outputs of the control logic (6), these data corresponding to the instantaneous logic state of the control logic (6), and the means (17,18,20) for the acquisition of the data being activated substantially simultaneously with the transmission means (11,13),
the transmission means (11,13) additionally exhibit a clock generator (16), which repetitively activates the transmission means (11,13) and the means (17,18,20) for the acquisition of the data at the instants which are specified by the clock generator (16) and which are periodically successive, the frequency of the clock generator (16) being higher than the change-over frequency of the logic state of the control logic (6).

2. Apparatus according to Claim 1, which apparatus possesses a cyclic mode of operation which is implemented in successive apparatus cycles, characterized in that the clock generator (16) repeatedly activates the means (11,13) for the transmission of data during an apparatus cycle.

3. Apparatus according to one of Claims 1 to 2, characterized in that the means (11,13) for the transmission of data transmit the data serially.

4. Apparatus according to one of Claims 1 to 3, characterized in that the apparatus (1) is designed as cardiac pacemaker, in which the means (3,4,4a,4b,4c) for the detection of physiological events monitor the electrical activity of the heart (2) and the means (3,8) for the stimulation of physiological processes stimulate the heart (2).

5. Apparatus according to one of Claims 1 to 4, characterized in that the means (3,4,4a,4b,4c) for the detection of physiological events form an electrical signal which corresponds to the associated physiological function and which is fed to an analog/digital converter (24), the digital output signal of which is fed to the means (11,13) for the transmission of data, which transmit said signal to the receiver (12).

6. Apparatus according to one of Claims 1 to 5, characterized in that it is possible to feed to the means (11,13) for the transmission of data, besides data concerning the logic state of the control logic (6), further data concerning the operational state of the apparatus (1), which the means (11,13) for the transmission of data transmit to the receiver (12).

7. Apparatus according to Claim 5 or 6, characterized in that the means (11,13) for the transmission of data transmit both the data corresponding to the logic state of the control logic (6) and also the digital output signals of the analog/digital converter (24) or the data concerning the operational state of the apparatus (1) in the form of a single data word (D0 to D9), in each instance a cohesive bit sequence (D0 to D3 or respectively D4 to D9) of the data word (D0 to D9) including the data corresponding to the logic state of the control logic (6) and the digital output signals of the analog/digital converter (24) or the data concerning the operational state of the apparatus (1).

## Revendications

1. Appareil médical (1) implantable dans le corps d'un être vivant, comportant
des moyens (3, 4, 4a, 4b, 4c) de détection de phénomènes physiologiques et/ou des moyens (3, 8) de stimulation de processus physiologiques,
une logique de commande (6), qui est munie de sorties et à laquelle sont raccordés les moyens de détection (3, 4, 4a, 4b, 4c) et/ou les moyens de stimulation (3, 8), et
des moyens (11, 13) de transmission télémétrique de données concernant l'état logique de la logique de commande (6) à un récepteur externe (12), les moyens (11, 13) de transmission de données étant reliés à la logique de commande (6),
caractérisé par le fait que
les moyens (11, 13) de transmission de données comportent des moyens (17, 18, 20) de détection des données présentes sur les sorties de la logique de commande (6), ces données correspondant à l'état logique instantané de la logique de commande (6), les moyens (17, 18, 20) de détection des données étant activés sensiblement en même temps que les moyens de transmission (11, 13),
les moyens de transmission (11, 13) comportent en outre un générateur de cadence (16), qui active de façon répétitive les moyens de transmission (11, 13) et les moyens (17, 18, 20) de détection des données aux instants fixés par le générateur de cadence (16) et qui se succèdent périodiquement, la fréquence du générateur de cadence (16) étant supérieure à la fréquence d'inversion de l'état logique de la logique de commande (6).

2. Appareil suivant la revendication 1, qui possède un fonctionnement cyclique se déroulant suivant des cycles successifs de l'appareil, caractérisé par le fait que le générateur (16) active plusieurs fois les moyens (11, 13) de transmission de données, pendant un cycle de l'appareil.

3. Appareil suivant l'une des revendications 1 et 2, caractérisé par le fait que les moyens (11, 13) de transmission de données transmettent les données en série.

4. Appareil suivant l'une des revendications 1 à 3, caractérisé par le fait que l'appareil (1) est réalisé sous la forme d'un stimulateur cardiaque, les moyens (3, 4, 4a, 4b, 4c) de détection de phénomènes physiologiques contrôlant l'activité électrique du coeur (2), tandis que les moyens (3, 8) de stimulation de phénomènes physiologiques stimulent le coeur (2).

5. Appareil suivant l'une des revendications 1 à 4, caractérisé par le fait que les moyens (3, 4, 4a, 4b, 4c) de détection de phénomènes physiologiques forment un signal électrique, qui correspond à la fonction physiologique associée et qui est envoyé à un convertisseur analogique/numérique (24), dont le signal de sortie numérique est envoyé aux moyens (11, 13) de transmission de données, qui transmettent ce signal au récepteur (12).

6. Appareil suivant l'une des revendications 1 à 5, caractérisé par le fait qu'aux moyens (11, 13) de transmission des données autres que les données concernant l'état logique de la logique de commande (6) peuvent être envoyées d'autres données, qui concernent l'état de fonctionnement de l'appareil (1) et que les moyens (11, 13) de transmission de données transmettent au récepteur (12).

7. Appareil suivant la revendication 5 ou 6, caractérisé par le fait que les moyens (11, 13) de transmission de données transmettent aussi bien les données qui correspondent à la logique de commande (6), que les signaux numériques du convertisseur analogique/numérique (24) ou les données, qui concernent l'état de fonctionnement de l'appareil (1), sous la forme d'un seul mot de données (D0 à D9), une suite binaire continue (D0 à D3 ou D4 à D9) du mot de données (D0 à D9) contenant des données qui correspondent à l'état logique de la logique de commande (6) et les signaux de sortie numériques du convertisseur analogique/numérique (24) ou les données qui concernent l'état de fonctionnement de l'appareil (1).
